(19) Europäisches Patentamt
European Patent Office
Office européen des brevets

(11) EP 1 056 694 B1

(12) EUROPEAN PATENT SPECIFICATION

(45) Date of publication and mention of the grant of the patent:
**07.01.2004 Bulletin 2004/02**

(21) Application number: **99904440.7**

(22) Date of filing: **29.01.1999**

(51) Int Cl.[7]: **C07C 15/08**, C07C 2/86, B01J 29/04

(86) International application number:
**PCT/US1999/001961**

(87) International publication number:
**WO 1999/038823 (05.08.1999 Gazette 1999/31)**

(54) **REACTIVE DISTILLATION PROCESS FOR THE PRODUCTION OF XYLENES**

REAKTIV DISTILLIERUNGSVERFAHREN ZUR HERSTELLUNG VON XYLOLEN

PROCEDE DE PRODUCTION DE XYLENES PAR DISTILLATION REACTIVE

(84) Designated Contracting States:
**DE ES FR GB IT NL**

(30) Priority: **30.01.1998 US 73158 P**

(43) Date of publication of application:
**06.12.2000 Bulletin 2000/49**

(73) Proprietor: **ExxonMobil Chemical Patents Inc.**
**Baytown, TX 77520-5200 (US)**

(72) Inventors:
- **MOHR, Gary, D.**
  **League City, TX 77573 (US)**
- **SANCHEZ, Leonel E., 4 Calle 2-41 Zona 15**
  **Guatemala City (GT)**

(74) Representative: **Dew, Melvyn John et al**
**ExxonMobil Chemical Europe Inc.**
**1830 Machelen (BE)**

(56) References cited:
**US-A- 4 002 697**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).



Printed by Jouve, 75001 PARIS (FR)

## Description

The present invention relates to a process for the production of xylenes by reactive distillation of toluene and a methylating agent.

### BACKGROUND OF THE INVENTION

Xylenes are important chemicals and find wide and varied application. Of the xylene isomers, i.e., ortho-, meta- and paraxylene, paraxylene is of particular value as a chemical intermediate in the manufacture of terephthalic acid, which is an intermediate used in the manufacturer of synthetic fibers. Various processes are used to make paraxylene. One such process, as disclosed in US-A-4,097,543, involves the selective production of paraxylene by the disproportionation of toluene in the presence of a coke-selectivated ZSM-5 catalyst to produce one mole of xylenes and one mole of benzene. Because of current and future anticipated environmental regulations involving benzene, it is desirable that paraxylene production does not result in the formation of significant quantities of by-product benzene.

Another process for making paraxylene, as disclosed in US-A-4,002,697, involves the methylation of toluene in the presence of an aluminosilicate zeolite such as ZSM-5. In order to reduce the amount of undesirable methylating agent/methylating agent and methylating agent/xylene reactions, the process can be carried out within a fixed bed reactor and with an excess of toluene at temperatures exceeding 400°C. However, when the process is carried out at these temperatures, a considerable amount of undesirable by-products can also be formed. For instance, at reaction temperatures greater than 400°C, methanol can form considerable amounts of undesirable by-products such as dimethyl-ether, $C_9^+$ aromatics, and $C_5$-by-products. Also, carrying out toluene methylation at these temperatures can result in the disproportionation of toluene with the resultant production of benzene. If the temperature used to carry out the process in the fixed bed reactor is reduced to 400°C or less, the amount of toluene converted to xylenes can be substantially reduced.

The present invention is directed to a process for producing xylene by toluene methylation which results in high toluene to xylenes conversion with reduced by-product formation.

### SUMMARY OF THE INVENTION

In accordance with the present invention, there is provided a reactive distillation process for the production of xylenes which comprises contacting toluene with a methylating agent in the presence of a toluene methylation catalyst.

In another embodiment of the present invention, there is provided a process for selectively producing paraxylene in preference to metaxylene and orthoxylene by reactive distillation which process comprises contacting toluene with a methylating agent in a reaction/distillation column containing a zeolite catalyst.

In carrying out the process of the present invention, substantial amounts of xylenes, including paraxylene, are produced while at the same time the formation of undesirable by-products is reduced.

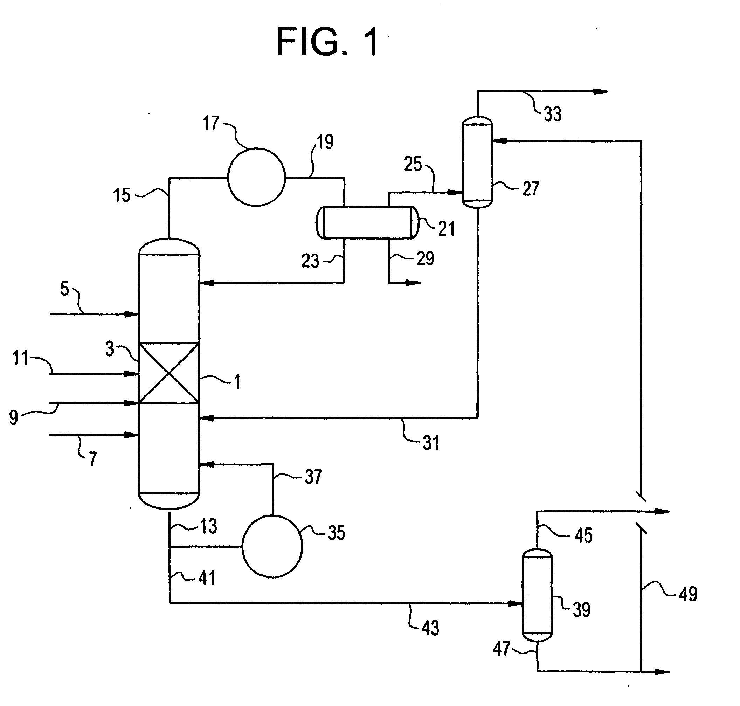

### BRIEF DESCRIPTION OF THE DRAWING

The Figure is a schematic diagram illustrating appropriate apparatus for use in carrying out the process of the present invention.

### DETAILED DESCRIPTION OF THE INVENTION

The term "reactive distillation", as used herein, means the production of xylenes while concurrently removing xylenes product from a reaction zone. The term "reaction/distillation column", as used herein, means a column in which toluene is contacted in the presence of a toluene methylation catalyst to produce xylenes and from which the xylenes are withdrawn.

In a preferred embodiment, the process of the present invention is carried out in a reaction/distillation column. In this embodiment, toluene and the methylating agent are usually fed continuously to the reaction/distillation column and the xylenes are continuously withdrawn from the bottom of the reaction/distillation column. In this embodiment, the continuous removal of xylenes from the reaction/distillation column increases the extent of reaction achieved within the column, thus providing very high toluene to xylenes conversion along with low by-product formation.

To explain more fully the invention, reference is now made to the Figure. Reaction/distillation column 1 is provided with reaction section 3, inlet for toluene feed 5, inlets for the methylating agent feed, 7, 9, and 11, column bottom outlet line 13, and column overhead outlet line 15. Condenser 17 is connected to overhead outlet line 15. Condenser outlet line 19 is connected to separator drum 21. Separator drum 21 is provided with exit line 23 through

which hydrocarbons are returned to reaction/distillation column 1. Separator drum overhead product take-off line 25 is connected to scrubber 27. Separator drum 21 is equipped with water take-off line 29. Scrubber 29 is provided with exit line 31 through which toluene and benzene can be returned to reaction/distillation column 1. Light gases exit through scrubber 27 through an overhead take-off line 33.

Reboiler 35 is provided with line 37 through which xylenes rich product can be returned to reaction/distillation column 1. Xylenes product not sent back to reaction/distillation column 1 is sent to fractionator 39 via line 41 and line 43. Fractionator 39 is provided with overhead line 45 where xylenes are removed. $C_9^+$ aromatics are removed via bottoms line 47 part of which is sent to scrubber 27 via line 49 to scrub $C_8$ hydrocarbons from the product gas.

In the operation of the process, the catalyst bed is preferably located in the enriching section of reaction/distillation column 1. Toluene is usually added through inlet 5 at the top of the catalyst bed. However, if a large amount of refluxed liquid is present in the reaction/distillation column, toluene can also be added through inlets located at the bottom of the catalyst bed. The toluene serves as both a reactant and a heat sink. As toluene trickles down the bed, the liquid toluene will vaporize due to the heat of reaction allowing close to isothermal operation within the reaction zone. Excess toluene is separated from the xylenes within the stripping section of the reaction/distillation column 1 and is returned to the reaction zone as vapor. Condenser 17 condenses most of the $C_6^+$ aromatics, which will usually be primarily toluene. All of the condensed liquids are then refluxed via line 23 to reaction/distillation column 1 except for water, which is drawn off from separator drum 21 as a separate stream via line 29. A small amount of vapor phase toluene may exit the separator drum 21 via line 25 with the uncondensed gases. This toluene may be recovered by scrubbing the light gases in scrubber 27 with a $C_6^+$ aromatic stream, which can be recycled back through line 31 to the stripping section of reaction/distillation column 1.

The methylating agent (e.g. methanol) is preferably injected into reaction/distillation column 1 via line 9 below the catalyst bed and preferably through a plurality of lines, e.g., lines 7, 9, and/or 11, within reaction/distillation column 1 at or near the reaction section containing the catalyst bed. Multiple injections can keep the methylating agent concentration low at points within the catalyst bed so as to reduce all reactions other than toluene methylation and to achieve nearly complete conversion of methanol. Methanol can be injected as either a vapor or liquid depending upon if methanol vaporization is desirable as a means of adsorbing heat released in the toluene methylation reaction. It is desirable to have substantially all of the methanol, e.g., 99 percent or greater, converted within the catalyst bed. If methanol is only partially converted, it can be separated out of the reaction/distillation column overhead stream along with dimethylether (DME) which may also be produced in small quantities. The overhead DME and methanol can then be recycled to extinction back to reaction/distillation column 1. The concentration of DME within the catalyst bed will eventually build to the point that it is catalytically consumed at the same rate as it is catalytically produced.

Reaction/distillation column 1 is preferably designed so that the pressure and temperature of the column is such that the reaction section will contain both liquid and gas phase xylenes and toluene. The concentration of xylenes within the reaction section will usually be much lower than the concentration of toluene in other sections of reaction/distillation column 1 because xylenes product is constantly removed from reaction/distillation column 1 while toluene is continuously fed to reaction section and refluxed. Liquid phase xylenes and toluene within the reaction section of the reaction/distillation column can enable coke precursors formed on the surface of the catalyst to be "washed away" thereby extending catalyst cycle length. Operating conditions and methylating agent injection points are preferably chosen so as to obtain high methylating agent conversion to xylenes. Since there should be low xylenes concentration within the reaction section, formation of undesirable by-products via xylenes transalkylation or xylenes methylation should be much lower than achievable in a fixed bed reactor at comparable process conditions.

The reaction/distillation column is preferably designed to condense most of the xylenes entering the top zone by contact/heat exchange with refluxed toluene. Preferably, the column is designed so that the catalyst bed operates nearly isothermally. Isothermal operations are beneficial as they serve to extend catalyst life and allow operations at an optimum reaction temperature. Since toluene methylation is exothermic, it usually will be necessary to remove reaction heat. This can be accomplished by vaporizing feed and refluxed toluene.

Catalysts suitable for use in the present invention include any catalysts that are effective for toluene methylation. Preferably, the catalyst used in the process of the present invention is a crystalline molecular sieve. Examples of exemplary catalysts are disclosed in US-A-3,965,207, 4,002,697, and 4,100,215, and W096/16004.

The preferred catalyst used in the process of the present invention is an intermediate pore size zeolite. Intermediate pore size zeolites have a pore size from about 5 to about 7 Å and include, for example, MFI, MEL, MFS, MEI, MTW, EUO, MTT, HEU, FER, and TON structure type zeolites. These zeolites and their isotopic framework structures are described in "Atlas of Zeolite Structure Types", eds. W. H. Meier, D.H. Olson, and Ch. Baerlocher, Elsevier, Fourth Edition, 1996. Examples of specific intermediate pore size zeolites include; AMS-1B, ZSM-5, ZSM-11, ZSM-12, ZSM-18, ZSM-22, ZSM-23, ZSM-34, ZSM-35, ZSM-38, ZSM-48, ZSM-50, and ZSM-57. Preferred zeolites are gallosilicate and aluminosilicate zeolites having an MFI structure.

The intermediate pore size zeolites will generally be a composition having the following molar relationship:

$$X_2O_{3:}:(n)\ Y0_2,$$

wherein X is a trivalent element such as aluminum, iron, boron, and/or gallium and Y is a tetravalent element such as silicon, tin, and/or germanium; and n has a value greater than 12, said value being dependent upon the particular type of zeolite. When the intermediate pore size zeolite is a MFI structure type zeolite, n is preferably greater than 10.

As known to persons skilled in the art, the acidity of a zeolite can be reduced using many techniques such as by steaming. In addition, the acidity of a zeolite is dependent upon the form of the zeolite with the hydrogen form having the highest acidity and other forms of the zeolite such as the sodium form having less acidity than the acid form. Accordingly, the mole ratios of silica to alumina and silica to gallia disclosed herein shall include not only zeolites having the disclosed mole ratios, but shall also include zeolites not having the disclosed mole ratios but having equivalent catalytic activity.

When the zeolite is an aluminosilicate zeolite, the zeolite will generally have a silica to alumina mole ratio from 10:1 to 700:1 and preferably from 20: 1 to 200:1.

When the zeolite is a gallium silicate zeolite, the zeolite usually will be a composition having the following molar relationship:

$$Ga_2O_3:ySiO_2$$

wherein y is between about 20 and about 500. The zeolite framework may contain only gallium and silicon atoms or may also contain a combination of gallium, aluminum, and silicon.

The zeolite catalyst can be incorporated with binder material resistant to the temperature and other conditions employed in hydrocarbon conversion processes such as refractory oxides and amorphous materials. Examples of suitable binder materials include synthetic or naturally occurring substances such as clays, silica, and/or metal oxides. Examples of such materials include silica, alumina, silica-alumina, magnesia, silica-magnesia, zirconia, silica-zirconia, silica-thoria, silica-beryllia, titania, silica-titania, ternary compositions, such as silica-alumina-thoria, silica-alumina-zirconia, silica-alumina-magnesia and silica-magnesia-zirconia, alumina sols, hydrated alumina, and clays such as bentonite or kaolin. The zeolite may also be composited with zeolitic material such as the zeolitic materials, which are disclosed in WO-96/16004.

When the catalyst used in the process of the present invention is a zeolite, the catalyst can be selectivated to enhance the amount of paraxylene produced by the process of the invention. The selectivation can be carried out either before the zeolite is loaded into the reactor or after loading the catalyst into the reactor. Examples of processes for selectivating catalysts include treating the surface of the catalyst with compounds of phosphorus and/or various metal oxides such as magnesium, antimony, and manganese.

Selectivation may also be accomplished by exposing the catalyst in a reactor bed to a thermally decomposable organic compound, e.g., toluene at a temperature in excess of the decomposition temperature of said compound, e. g., from about 400°C to about 650°C, more preferably 425°C to about 550°C, at a WHSV in the range of from about 0.0454 to about 9.07 kg of feed per kg of catalyst per hour (about 0.1 to about 20 lbs of feed per pound of catalyst per hour), at a pressure in the range of from about 101.3 kPa to about 10.13 MPa (about 1 to about 100 atmospheres), and in the presence of 0 to about 2 moles of hydrogen, more preferably from about 0.1 to about 1 moles of hydrogen per mole of organic compound, and optionally in the presence of 0 to about 10 moles of nitrogen or another inert gas per mole of organic compound. This process is conducted for a period of time until a sufficient quantity of coke has deposited on the catalyst surface, generally at least about 2% by weight and more preferably from about 8 to about 40% by weight of coke.

Selectivation of the catalyst may also be accomplished using organosilicon compounds. The silicon compounds may comprise a polysiloxane including silicones, a siloxane, and a silane including disilanes and alkoxysilanes.

Silicone compounds that can be used in the present invention

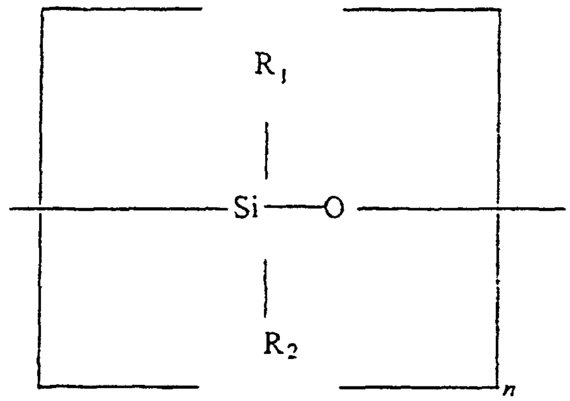

wherein $R_1$ is hydrogen, fluoride, hydroxy, alkyl, aralkyl, alkaryl or fluoro-alkyl. The hydrocarbon substituents generally contain from 1 to about 10 carbon atoms and preferably are methyl or ethyl groups. $R_2$ is selected from the same group as $R_1$, and n is an integer of at least 2 and generally in the range of 2 to about 1000. The molecular weight of the silicone compound employed is generally between about 80 to about 20,000 and preferably about 150 to about 10,000. Representative silicone compounds include dimethylsilicone, diethylsilicone, phenylmethylsilicone, methyl hydrogensilicone, ethylhydrogensilicone, phenylhydrogensilicone, fluoropropylsilicone, ethyltrifluoropropylsilicone, tetrachlorophenyl methyl methylethylsilicone, phenylethylsilicone, diphenylsilicone, methyltri silicone, tetrachlorophenylethyl silicone, methylvinylsilicone and ethylvinylsilicone. The silicone compound need not be linear but may be cyclic as for example hexamethylcyclotrisiloxane, octamethylcyclotetrasiloxane, hexaphenyl cyclotrisiloxane and octaphenylcyclotetrasiloxane. Mixtures of these compounds may also be used as well as silicones with other functional groups.

Useful siloxanes and polysiloxanes include as non-limiting example hexamethylcyclotrisiloxane, occamethylcyclotetrasiloxane, decamethyl cyclopentasiloxane, hexamethyldisiloxane, octamethytrisiloxane, decamethyltetrasiloxane, hexaethylcyclotrisiloxane, octaethylcyclo tetrasiloxane, hexaphenylcyclotrisiloxane and octaphenylcyclo-tetrasiloxane.

Useful silanes, disilanes, or alkoxysilanes include organic substituted silanes having the general formula:

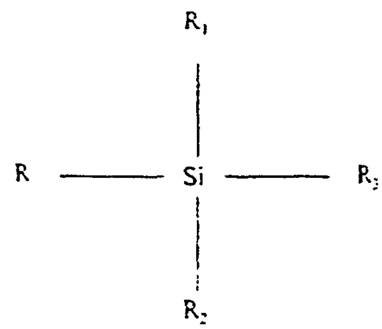

wherein R is a reactive group such as hydrogen, alkoxy, halogen, carboxy, amino, acetamide, trialkylsilyloxy, $R_1$, $R_2$ and $R_3$ can be the same as R or can be an organic radical which may include alkyl of from 1 to about 40 carbon atoms, alkyl or aryl carboxylic acid wherein the organic portion of alkyl contains 1 to about 30 carbon atoms and the aryl group contains about 6 to about 24 carbons which may be further substituted, alkylaryl and arylalkyl groups containing about 7 to about 30 carbon atoms. Preferably, the alkyl group for an alkyl silane is between about 1 and about 4 carbon atoms in chain length. Mixtures may also be used.

The silanes or disilanes include, as non-limiting examples, dimethylphenylsilane, phenyltrimethylsilane, triethylsilane and hexamethyldisilane. Useful alkoxysilanes are those with at least one silicon-hydrogen bond.

The process of the present invention is typically operated at a temperature no greater than about 320°C and, preferably, from about 225°C to about 300°C. However, the particular temperature is dependent upon a number of factors including, for example, the reactant flow rates, the operating pressure and the desired production rate. The molar ratio of toluene to methylating agent is preferably greater than 1 and, more preferably, greater than about 10.

Hydrogen gas can be supplied to the reaction as an anticoking agent and diluent.

Typical methylating agents include methanol, dimethylether, methylchloride, methylbromide, methylcarbonate, acetaldehyde, dimethoxyethane, acetone, and dimethylsulfide. One skilled in the art will know that other methylating agents may be employed in the process of this invention based on the description provided therein. Preferred methylating agents are methanol and dimethylether.

Example

The following example is a computer simulation of the process of the present invention. Simulation results were obtained using PROII Version 4.18 Software from Simulation Services Inc. Vapor pressure measurements were converted into equilibrium vapor and liquid compositions using an activity coefficient model, i.e., the Non-Random, Two-Liquid (NRTL) method.

Reaction stoichiometry used for the simulation was the following:

$$7 \text{ Methanol} + 5 \text{ Toluene} \rightarrow 1 \text{ Ethylene} + 5 \text{ Xylene} + 7 \text{ } H_2O$$

In the process simulation, the reaction proceeds in a reaction/distillation column similar to reaction/distillation column (1) shown in the Figure. A fifty theoretical distillation stage reaction/distillation column was used in the simulation. The distillation stages in the example are identified by their order from top to bottom of the reaction/distillation column. Thus, the top stage is Stage 1 while the bottom stage is Stage 50. Stages between Stage 1 and stage 50 are identified using this numbering sequence. The toluene methylation catalyst used for the simulation is located in Stages 21 through 24 (below the toluene feed [Stage 20] and above the methanol feed [Stage 25]. In the simulation, it is assumed the catalyst will convert 100% of the methanol.

The methanol is fed to Stage 25 of the reaction/distillation column at a rate of 400 kg/hr and at a temperature of 140°C. The toluene is fed to Stage 20 of the reaction/distillation column at a rate of 1000 kg/hr and at a temperature of 190°C. The pressure of the reaction/distillation column is 7.4 kg/cm$^2$ (105 psia). The temperature in the reaction zone is about 190°C. A reboiler and condenser are connected to the reaction/distillation column similar to the connections to reboiler and condenser shown in the Figure. The heat input of the reboiler was set at 16742 kJ/hr (4000 kcal/hr) and the condenser duty was determined to be -16930 kJ/hr (-4045 kcal/hr).

Table I below provides summarizes the stream component flow rates:

Table I

| Flow, kg/hr | Methanol Feed | Toluene Feed | Overhead Prod.* | Bottoms Prod ** |
|---|---|---|---|---|
| Ethylene | 0 | 0 | 50.0 | 0 |
| Methanol | 400 | 0 | 0 | 0 |
| Toluene | 0 | 1000 | 51.0 | 127.4 |
| Xylene | 0 | 0 | 0 | 946.7 |
| Water | 0 | 0 | 224.9 | 0 |
| Total | 400 | 1000 | 325.9 | 1074.1 |

* Similar to stream take-off at line 25 shown in Figure. No water is taken off at line 29.

** Similar to stream take-off at line 41 shown in Figure.

**Claims**

1. A process for making xylenes which comprises reacting toluene with a methylating agent under reactive distillation conditions and in the presence of a toluene methylation catalyst.

2. The process recited in Claim 1, wherein the process is carried out in a reaction/distillation column.

3. The process recited in Claims 1 or 2, wherein 99 % or greater of the methylating agent is consumed.

4. The process recited in any preceding claim, wherein the process is carried out at a temperature no greater than 320°C.

5. The process recited in any preceding claim, wherein the molar ratio of toluene to methylating agent is greater than 10:1.

6. The process recited in Claims 2 to 5, wherein the reaction/distillation column contains a reactor section and the concentration of the methylating agent in the reactor section is less than the other sections of the column.

7. The process recited in any preceding claim, wherein the methylating agent is methanol, dimethylether, or mixtures thereof.

8. A process for producing xylenes from toluene as recited in Claims 2 to 7, wherein the process comprises:

   (a) contacting toluene with a methylating agent in a reaction zone located in a reaction/distillation column which is maintained at methylating conditions and contains a toluene methylation catalyst; and,
   (b) recovering the xylenes.

9. The process recited in Claims 2 to 8, wherein the methylating agent is injected continuously into the reaction/distillation column.

10. The process recited in Claims 2 to 8, wherein the methylating agent is injected into the reaction/distillation column at a plurality of injection points.

11. The process recited in Claims 2 to 10, wherein the xylenes are recovered from a lower end of the reaction/distillation column.

12. The process recited in Claims 8 to 11, wherein the temperature in the reaction zone is from 225°C to 300°C.

13. The process recited in Claims 8 to 12, wherein the concentration of methylating agent in the reaction zone is less than other. sections of the reaction/distillation column.

14. The process recited in any preceding claim, wherein the methylating agent is methanol.

15. The process recited in Claims 8 to 14, wherein the reaction zone contains liquid and gas phase xylenes and toluene.

16. The process recited in Claims 2 to 15, wherein xylenes product is continuously removed from the reaction/distillation column.

17. The process recited in Claims 2 to 16, wherein toluene is continuously fed to the reaction/distillation column and refluxed.

18. The process recited in any preceding claim, wherein the toluene methylation catalyst is a zeolite catalyst.

19. The process recited in Claim 18, wherein the zeolite is an intermediate pore size zeolite.

20. The process recited in Claim 19, wherein the zeolite has a structure type selected from the group consisting of MFI, MEL, MTW, MTT, FER, TON, and EUO.

21. The process recited in Claim 19 or 20, wherein the zeolite has a composition with the following molar relationship:

$$X_2O_3:(n)\ YO_2,$$

wherein X is aluminum, boron, iron, and/or gallium, Y is silicon, tin, and/or germanium, and n has a value greater than 10.

22. The process recited in Claims 18 to 21, wherein the zeolite is an aluminosilicate zeolite or a gallosilicate zeolite.

23. The process recited in Claim 22, wherein the zeolite has a silica to alumina mole ratio of from 20:1 to 700:1 or a silica to gallia mole ratio from 20:1 to 500:1.

24. The process recited in Claims 21 to 23, wherein the zeolite has a MFI, MEL, MTT or TON structure type.

25. The process recited in Claims 18 to 24, wherein the catalyst is preselectivated.

26. The process recited in Claim 25, wherein the catalyst is preselectivated with coke.

27. The process created in Claim 25, wherein the catalyst is selectivated with an organosilicon compound.

28. The process recited in Claims 18 to 27, wherein the catalyst further comprises a zeolitic binder.

## Patentansprüche

1. Verfahren zur Herstellung von Xylolen, bei dem Toluol mit Methylierungsmittel unter reaktiven Destillationsbedingungen und in Gegenwart von Toluolmethylierungskatalysator umgesetzt wird.

2. Verfahren nach Anspruch 1, bei dem das Verfahren in einer Reaktions-/Destillationskolonne durchgeführt wird.

3. Verfahren nach Anspruch 1 oder 2, bei dem 99 % oder mehr des Methylierungsmittels verbraucht werden.

4. Verfahren nach einem der vorhergehenden Ansprüche, bei dem das Verfahren bei einer Temperatur von nicht größer als 320 °C durchgeführt wird.

5. Verfahren nach einem der vorhergehenden Ansprüche, bei dem das Molverhältnis von Toluol zu Methylierungsmittel größer als 10 : 1 ist.

6. Verfahren nach einem der Ansprüche 2 bis 5, bei dem die Reaktions-/Destillationskolonne einen Reaktorabschnitt enthält und die Konzentration des Methylierungsmittels in dem Reaktorabschnitt geringer als in den anderen Abschnitten der Kolonne ist.

7. Verfahren nach einem der vorhergehenden Ansprüche, bei dem das Methylierungsmittel Methanol, Dimethylether oder Mischungen derselben ist.

8. Verfahren zur Herstellung von Xylolen aus Toluol nach einem der Ansprüche 2 bis 7, bei dem

   (a) Toluol mit Methylierungsmittel in einer Reaktionszone kontaktiert wird, die in einer Reaktions-/Destillationskolonne angeordnet ist, die auf Methylierungsbedingungen gehalten wird und Toluolmethylierungskatalysator enthält und

   (b) die Xylole gewonnen werden.

9. Verfahren nach einem der Ansprüche 2 bis 8, bei dem das Methylierungsmittel kontinuierlich in die Reaktions-/Destillationskolonne injiziert wird.

10. Verfahren nach einem der Ansprüche 2 bis 8, bei dem das Methylierungsmittel in die Reaktions-/Destillationskolonne an einer Vielzahl von Injektionsstellen injiziert wird.

**11.** Verfahren nach einem der Ansprüche 2 bis 10, bei dem die Xylole aus einem unteren Ende der Reaktions-/Destillationskolonne gewonnen werden.

**12.** Verfahren nach einem der Ansprüche 8 bis 11, bei dem die Temperatur in der Reaktionszone 225 °C bis 300 °C beträgt.

**13.** Verfahren nach einem der Ansprüche 8 bis 12, bei dem die Konzentration von Methylierungsmittel in der Reaktionszone geringer als in anderen Abschnitten der Reaktions-/Destillationskolonne ist.

**14.** Verfahren nach einem der vorhergehenden Ansprüche, bei dem das Methylierungsmittel Methanol ist.

**15.** Verfahren nach einem der Ansprüche 8 bis 14, bei dem die Reaktionszone Flüssig- und Gasphasenxylole und -toluol enthält.

**16.** Verfahren nach einem der Ansprüche 2 bis 15, bei dem das Produkt an Xylolen kontinuierlich aus der Reaktions-/Destillationskolonne entfernt wird.

**17.** Verfahren nach einem der Ansprüche 2 bis 16, bei dem Toluol kontinuierlich in die Reaktions-/Destillationskolonne eingeführt und refluxiert wird.

**18.** Verfahren nach einem der vorhergehenden Ansprüche, bei dem der Toluolmethylierungskatalysator ein Zeolithkatalysator ist.

**19.** Verfahren nach Anspruch 18, bei dem der Zeolith ein Zeolith mit mittlerer Porengröße ist.

**20.** Verfahren nach Anspruch 19, bei dem der Zeolith einen Strukturtyp aufweist, der aus der Gruppe bestehend aus MFI, MEL, MTW, MTT, FER, TON und EUO ausgewählt ist.

**21.** Verfahren nach Anspruch 19 oder 20, bei dem der Zeolith eine Zusammensetzung mit der folgenden molaren Beziehung aufweist:

$$X_2O_3 : (n)YO_2,$$

in der X Aluminium, Bor, Eisen und/oder Gallium ist, Y Silicium, Zinn und/oder Germanium ist und n einen Wert von größer als 10 aufweist.

**22.** Verfahren nach einem der Ansprüche 18 bis 21, bei dem der Zeolith ein Aluminosilikatzeolith oder ein Gallosilikatzeolith ist.

**23.** Verfahren nach Anspruch 22, bei dem der Zeolith ein Molverhältnis von Siliciumdioxid zu Aluminiumoxid von 20 : 1 bis 700 : 1 oder ein Molverhältnis von Siliciumdioxid zu Galliumoxid von 20 : 1 bis 500 : 1 aufweist.

**24.** Verfahren nach einem der Ansprüche 21 bis 23, bei dem der Zeolith einen MFI-, MEL-, MTT- oder TON-Strukturtyp aufweist.

**25.** Verfahren nach einem der Ansprüche 18 bis 24, bei dem der Katalysator vorselektiviert wird.

**26.** Verfahren nach Anspruch 25, bei dem der Katalysator mit Koks vorselektiviert wird.

**27.** Verfahren nach Anspruch 25, bei dem der Katalysator mit einer Organosiliciumverbindung selektiviert wird.

**28.** Verfahren nach einem der Ansprüche 18 bis 27, bei dem der Katalysator ferner ein Zeolithbindemittel umfasst.

## Revendications

1. Procédé pour la production de xylènes, qui comprend la réaction de toluène avec un agent de méthylation dans des conditions de distillation réactives et en présence d'un catalyseur de méthylation du toluène.

2. Procédé suivant la revendication 1, ledit procédé étant mis en oeuvre dans une colonne de réaction/distillation.

3. Procédé suivant la revendication 1 ou 2, dans lequel une proportion égale ou supérieure à 99% de l'agent de méthylation est consommée.

4. Procédé suivant l'une quelconque des revendications précédentes, qui est mis en oeuvre à une température non supérieure à 320°C.

5. Procédé suivant l'une quelconque des revendications précédentes, dans lequel le rapport molaire du toluène à l'agent de méthylation est supérieur à 10:1.

6. Procédé suivant les revendications 2 à 5, dans lequel la colonne de réaction/distillation contient une section de réacteur et la concentration de l'agent de méthylation dans la section de réacteur est inférieure à celle dans les autres sections de la colonne.

7. Procédé suivant l'une quelconque des revendications précédentes, dans lequel l'agent de méthylation est le méthanol, l'éther diméthylique ou leurs mélanges.

8. Procédé pour la production de xylènes à partir de toluène suivant les revendications 2 à 7, ledit procédé comprenant les étapes consistant :

   (a) à mettre en contact du toluène avec un agent de méthylation dans une zone réactionnelle située dans une colonne de réaction/distillation qui est maintenue dans des conditions de méthylation et qui contient un catalyseur de méthylation du toluène ; et
   (b) à recueillir les xylènes.

9. Procédé suivant les revendications 2 à 8, dans lequel l'agent de méthylation est injecté de manière continue dans la colonne de réaction/distillation.

10. Procédé suivant les revendications 2 à 8, dans lequel l'agent de méthylation est injecté dans la colonne de réaction/distillation à une pluralité de points d'injection.

11. Procédé suivant les revendications 2 à 10, dans lequel les xylènes sont recueillis à une extrémité inférieure de la colonne de réaction/distillation.

12. Procédé suivant les revendications 8 à 11, dans lequel la température dans la zone réactionnelle est comprise dans l'intervalle de 225°C à 300°C.

13. Procédé suivant les revendications 8 à 12, dans lequel la concentration d'agent de méthylation dans la zone réactionnelle est inférieure à celle dans les autres sections de la colonne de réaction/distillation.

14. Procédé suivant l'une quelconque des revendications prédédentes, dans lequel l'agent de méthylation est le méthanol.

15. Procédé suivant les revendications 8 à 14, dans lequel la zone réactionnelle contient des xylènes et du toluène en phase liquide et en phase gazeuse.

16. Procédé suivant les revendications 2 à 15, dans lequel le produit consistant en xylènes est évacué de manière continue de la colonne de réaction/distillation.

17. Procédé suivant les revendications 2 à 16, dans lequel le toluène est introduit de manière continue dans la colonne de réaction/distillation et est soumis à un reflux.

**18.** Procédé suivant l'une quelconque des revendications précédentes, dans lequel le catalyseur de méthylation du toluène est un catalyseur zéolitique.

**19.** Procédé suivant la revendication 18, dans lequel la zéolite est une zéolite ayant un diamètre intermédiaire des pores.

**20.** Procédé suivant la revendication 19, dans lequel la zéolite a un type de structure choisi dans le groupe consistant en MFI, MEL, MTW, MTT, FER, TON et EUO.

**21.** Procédé suivant la revendication 19 ou 20, dans lequel la zéolite a une composition ayant la relation molaire suivante:

$$X_2O_3{:}(n)YO_2$$

dans laquelle X représente l'aluminium, le bore, le fer et/ou le gallium, Y représente le silicium, l'étain et/ou le germanium, et n a une valeur supérieure à 10.

**22.** Procédé suivant les revendications 18 à 21, dans lequel la zéolite est une zéolite du type aluminosilicate ou une zéolite du type gallosilicate.

**23.** Procédé suivant la revendication 22, dans lequel la zéolite a un rapport molaire de la silice à l'alumine compris dans l'intervalle de 20:1 à 700:1 ou un rapport molaire de la silice à l'oxyde de gallium compris dans l'intervalle de 20:1 à 500:1.

**24.** Procédé suivant les revendications 21 à 23, dans lequel la zéolite a un type de structure MFI, MEL, MTT ou TON.

**25.** Procédé suivant les revendications 18 à 24, dans lequel le catalyseur est préalablement rendu sélectif.

**26.** Procédé suivant la revendication 25, dans lequel le catalyseur est préalablement rendu sélectif avec du coke.

**27.** Procédé suivant la revendication 25, dans lequel le catalyseur est rendu sélectif avec un composé organique de silicium.

**28.** Procédé suivant les revendications 18 à 27, dans lequel le catalyseur comprend en outre un liant zéolitique.

FIG. 1